# EUROPÄISCHE PATENTSCHRIFT

(11) **EP 2 055 240 B1**
(45) Veröffentlichungstag und Bekanntmachung des Hinweises auf die Patenterteilung: **01.08.2012**
(21) Anmeldenummer: 08167424.4
(22) Anmeldetag: 23.10.2008
(51) Int. Cl.: A61B 17/06

(54) **Nahtaufsatz für ein chirurgisches Nahtinstrument**
Stitch section for a surgical stitching instrument
Chapeau de suture pour un instrument chirurgical de suture

(30) Priorität: 25.10.2007 DE 102007052185
(43) Veröffentlichungstag der Anmeldung: 06.05.2009
(73) Patentinhaber: Karl Storz GmbH & Co. KG, 78532 Tuttlingen (DE)
(72) Erfinder: Kreidler, Bodo, 78532 Tuttlingen (DE)
(74) Vertreter: Witte, Weller & Partner

(56) Entgegenhaltungen:
- DE-A1-102004 024 188
- US-A- 4 103 690
- US-A- 5 769 862
- US-A1- 2005 033 365

## Beschreibung

Die Erfindung betrifft einen Nahtaufsatz für ein chirurgisches Nahtinstrument mit einem proximalseitigen Anschluss zum Verbinden mit dem Nahtinstrument, mit einem hohlschaftartigen Körper, der distalseitig eine gekrümmte Hohlnadel aufweist, wobei ein Faden oder ein Fadenbündel durch den Nahtaufsatz führbar ist und ein Querschnitt der gekrümmten Hohlnadel ovalär ist.

Solch ein Nahtaufsatz ist aus der US 2005/0033365 A1 bekannt.

Dieser wird für Operationen an der Rotatorenmanschette verwendet, weist dabei zumeist eine Hohlnadel mit einem runden Querschnitt auf, der aber auch oval sein kann.

In der US 4,103,690 wird ein Anschluss für einen Herzschrittmacher beschrieben, an dem eine Hohlnadel so angeordnet ist, dass damit ein Anordnen an eine gewünschte Anschlussstelle im Herzbereich ermöglicht wird. Diese Hohlnadel ist dazu im Querschnitt rund ausgestaltet, und ist an einem Ende so schräg geschnitten, dass eine ovale Öffnung resultiert.

Ein ähnlicher Nahtaufsatz sowie ein chirurgisches Nahtinstrument, das mit dem Nahtaufsatz verbunden werden soll, ist aus dem Katalog "Arthroskopie, Sportmedizin, Wirbelsäulen-Chirurgie", 2. Ausgabe 1/2005, Seite 106 (Blatt ART-SHF 14A) der Karl Storz GmbH & Co. KG bekannt. Dieses chirurgische Nahtinstrument weist einen etwa stabförmigen Körper auf, durch den ein chirurgischer Faden von proximal nach distal zu einem distalen Nahtaufsatz geführt ist. Im Körper des Nahtinstruments ist eine Mulde ausgespart, längs der ein Abschnitt des Fadens freiliegend geführt ist.

Dieses chirurgische Nahtinstrument kann mit unterschiedlichen Nahtaufsätzen, die sich insbesondere durch unterschiedlich gebogene und ausgeformte gekrümmte Hohlnadeln unterscheiden, verbunden werden.

Dazu weist der Nahtaufsatz einen hohlschaftartigen Körper auf, der proximal einen Anschluss zum Verbinden mit den chirurgischen Nahtinstrument aufweist. Der chirurgische Faden oder das Fadenbündel, das durch den Körper des chirurgischen Nahtinstrumentes hindurchgeführt ist, wird auch durch den Nahtaufsatz hindurchgeführt und tritt an der Spitze der gekrümmten Hohlnadel aus. Bei chirurgischen Fäden, die als relativ steifer, jedoch flexibler Körper ausgebildet sind, stellt es kein großes Problem dar, den Faden durch die gekrümmte oder manchmal gar gewundene Hohlnadel durchzuschieben, bis dieser am distalen Ende im Bereich der Spitze der Hohlnadel austritt. Allerdings muss der Faden eine gewisse Steifigkeit in Längsrichtung aufweisen, damit er durch die relativ lange Wegstrecke des Nahtaufsatzes, etwa 150 mm, hindurchgeschoben werden kann. Dazu hat sich etabliert, das Lumen der Hohlnadel mit einem kreisförmigen Querschnitt auszustatten, der in etwa dem Querschnitt des Fadens entspricht.

In der Weiterentwicklung der Nähtechnik wurde insbesondere bei Nahtmaterialien, die zum Aufspleißen neigen, das distale Ende mit einem relativ steifen Endabschnitt versehen, bspw. dadurch, dass auf dieses Ende eine Hülse aufgepresst wird oder der Endbereich verklebt wird. Bei manchen Nähtechniken soll der Faden nicht aus einem Monofilament, sondern aus einem Multifilament bestehen, wobei zur Handhabung es notwendig ist, die Enden der Multifilamente über eine solche steife Hülse miteinander zu verbinden oder zu verkleben.

Bei gekrümmten Nadeln, insbesondere bei sehr stark gekrümmten Nadeln, ist es nunmehr nicht mehr möglich, die steifen, ggf. mehrere Zentimeter langen Endabschnitte durch einen Hohlkörper zu schieben, dessen Lumen in etwa dem Außendurchmesser entspricht. Der versteifte Endabschnitt würde sich in einer solchen Krümmung festklemmen oder verkanten und könnte nicht mehr nach distal geschoben werden.

Daher müssen relativ großlumige Hohlnadeln vorgesehen werden, um dem steifen Endabschnitt genügend Raum zur Verfügung zu stellen, damit diese sich um die Krümmung bewegen kann. Dies resultiert in einer relativ dicken Nadel, die dementsprechend eine großflächige Einstichstelle im Gewebe zur Durchführung des Nähvorganges verursacht.

Dies steht aber im Gegensatz zu dem allgemeinen Bestreben in der Chirurgie, einen solchen Vorgang möglichst wenig traumatisch durchzuführen.

Es ist daher Aufgabe der vorliegenden Erfindung, hier Abhilfe zu schaffen und einen Nahtaufsatz dahingehend weiterzuentwickeln, dass er bei möglichst kleiner Bauweise auch ein Durchschieben von Fäden mit einem versteiften Nahtfadenende ermöglicht.

Erfindungsgemäß wird die Aufgabe dadurch gelöst, dass der Querschnitt als Flachoval ausgebildet ist, dessen beide gegenüberliegende Seiten flach verlaufen, und dass sich eine längere Achse des Flachovales in einer Krümmungsebene der gekrümmten Hohlnadel erstreckt.

Diese Maßnahme hat den Vorteil, dass in dieser Erstreckung der längeren Achse des Ovals dem versteiften Fadenende die Möglichkeit gegeben wird, sich um eine Krümmung zu bewegen, ohne dass sich dieser steife Abschnitt verkantet oder verklemmt. Da dieser Raum ja nur in einer Ebene, nämlich in der Ebene der Krümmung, benötigt wird, reicht es aus, wenn das Lumen lediglich in diese Richtung vergrößert bzw. gestreckt wird, nämlich längs des längeren Durchmessers des Ovales. In der Querrichtung dazu, also längs der kürzeren Achse des Ovales, wird dieser Raum nicht benötigt, so dass das Lumen in dieser Richtung nicht erweitert werden muss.

Diese Maßnahme hat zusätzlich den erheblichen Vorteil, dass die gegenüberliegenden geradlinigen Wände des Flachovales sehr starke Druck- oder Zugkräfte aufnehmen können. Wie zuvor erwähnt, wirken ja auf die gekrümmte Hohlnadel, die selbst ein äußerst schlanker Körper im Bereich von einem Durchmesser von 1 bis 2 mm darstellt, relativ starke Kräfte beim Einstechvorgang ein. Durch die Widerstandskräfte beim Einstechen in ein Gewebe wirken Kräfte, die ein Aufweiten oder Begradigen der Krümmung verursachen könnten. Diese Kräfte können nun durch die Flachovalseiten gut aufgenommen werden, so dass im Ergebnis die Wandstärke sogar geringer gewählt werden kann als bei einer Nadel mit kreisförmigem Querschnitt.

Diese Maßnahme hat auch den erheblichen Vorteil, dass das resultierende Querschnittsvolumen wesentlich geringer ist als das Querschnittsvolumen einer Hohlnadel mit kreisförmigem Querschnitt, dessen Durchmesser der längeren Achse des ovalen Querschnitts entspricht. Dadurch verringert sich auch das Einstichvolumen, so dass dieser Vorgang wesentlich weniger traumatisch durchgeführt werden kann als mit einer Nadel mit rundem Querschnitt und mit dem Durchmesser der langen Achse.

Zugleich erlaubt die ovaläre Ausgestaltung das Ausbilden des Hohlkörpers der Nadel mit einer geringeren Wandstärke, denn die Geometrie des Ovales erlaubt, höhere Kräfte aufzunehmen, die in Richtung der längeren Achse wirken, als bei einem kreisförmigen Körper. Da beim Einstechen einer gekrümmten Nadel in ein Gewebe die Widerstandskräfte sich insbesondere auf den gekrümmten Bereich auswirken, erlaubt die ovale Geometrie, höhere Kräfte aufzunehmen, was daraus resultiert, dass man Hohlnadeln mit dünneren Wandstärken bauen kann, wenn diese den ovalären Querschnitt aufweisen.

Wie zuvor erwähnt, wird im Wesentlichen in der Krümmungsebene der Platz benötigt, um den versteiften Endabschnitt hindurchzuschieben. Mit dieser Ausrichtung des Ovals kann das Ende, ohne dass es verdreht oder verdrillt oder aus dieser Ebene abweichen muss, einfach und somit widerstandsarm durch den gekrümmten Bereich der Nadel hindurchgeschoben werden.

In einer weiteren Ausgestaltung der Erfindung entspricht die kurze Achse des ovalären Querschnittes in etwa dem Durchmesser des versteiften distalen Endes des Fadens oder des Fadenbündels.

Diese Maßnahme hat den Vorteil, dass in dieser Richtung der versteifte Endabschnitt seitlich geführt ist, so dass keine Verkantungen dieses Abschnitts beim Durchlaufen der gekrümmten Hohlnadel erfolgen können, sondern dieser Vorgang durch diese Seitenführung zielgerichtet geführt ablaufen kann.

Die Erfindung betrifft auch ein chirurgisches Nahtinstrument mit einem etwa stabförmigen Körper, durch den ein chirurgischer Faden oder ein Fadenbündel von proximal nach distal führbar ist, wobei der Körper mit einem Nahtaufsatz entsprechend der vorliegenden Erfindung verbunden ist.

Es versteht sich, dass die vorstehend genannten und die nachstehend noch zu erläuternden Merkmale nicht nur in der angegebenen Kombinationen, sondern auch in anderen Kombinationen oder in Alleinstellung einsetzbar sind, ohne den Rahmen der vorliegenden Erfindung zu verlassen.

Die Erfindung wird nachfolgend anhand eines ausgewählten Ausführungsbeispiels im Zusammenhang mit den beiliegenden Zeichnungen näher beschrieben und erläutert.

Es zeigen:
- Fig. 1: eine Explosionsansicht eines chirurgischen Nahtinstruments, das mit einem Nahtaufsatz verbunden werden kann, wobei dargestellt ist, wie ein chirurgischer Faden sowohl durch das chirurgische Nahtinstrument als auch durch den Nahtaufsatz hindurchgeführt ist,
- Fig. 2: eine stark vergrößerte ausschnittsweise Seitenansicht des distalen Endbereiches des Nahtaufsatzes mit der gekrümmten Hohlnadel,
- Fig. 3: eine um 90° um die Längsachse gedrehte Draufsicht auf den Bereich von Fig. 2,
- Fig. 4: einen Schnitt längs der Linie IV-IV in Fig. 2,
- Fig. 5: einen der Fig. 4 vergleichbaren Schnitt einer Hohlnadel mit kreisförmigem Querschnitt,
- Fig. 6: einer der Darstellung von Fig. 2 vergleichbaren Längsschnitt des distalen Endbereiches eines Nahtaufsatzes, wobei ein Faden mit einem versteiften distalen Fadenende eingeschoben ist,
- Fig. 7: eine der Darstellung von Fig. 6 vergleichbare Darstellung, wobei das versteifte Fadenende weiter in den Krümmungsbereich eingeschoben ist,
- Fig. 8: eine der Darstellung von Figuren 6 und 7 vergleichbare Darstellung, wobei das versteifte Fadenende gerade aus der Spitze der Nadel herausgeschoben wird, und
- Fig. 9: einen Schnitt längs der Linie IX-IX in Fig. 7.

Ein in den Figuren dargestellter Nahtaufsatz für ein chirurgisches Nahtinstrument ist in seiner Gesamtheit mit der Bezugsziffer 10 bezeichnet.

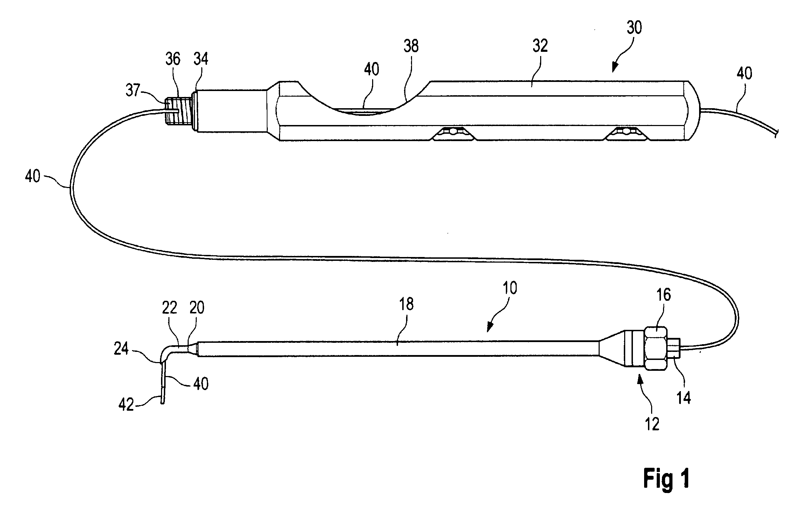

Wie insbesondere aus Fig. 1 zu entnehmen, weist der Nahtaufsatz 10 proximalseitig einen Anschluss 12 auf. Der Anschluss 12 weist einen nach proximal vorspringenden Zapfen 14 auf, der von einer Überwurfmutter 16 umgeben ist. Vom Anschluss 12 erstreckt sich ein dünner hohlschaftförmiger Körper 18 mit einem Außendurchmesser von etwa 2 mm und einer Länge von bspw. 150 mm. Am distalen Ende 20 geht der Körper 18 in eine gekrümmte Hohlnadel 22 über, die in einer angeschrägten Spitze 24 endet, wie das bei Nadeln oder Spritzen, die in ein Gewebe eingestochen werden sollen, üblich ist.

Der Nahtaufsatz 10 ist dazu vorgesehen, mit einem chirurgischen Nahtinstrument verbunden zu werden, das in Fig. 1 in seiner Gesamtheit mit der Bezugsziffer 30 bezeichnet ist.

Das chirurgische Nahtinstrument 30 weist einen etwa stabförmigen Körper 32 auf, der als Handgriff ausgebildet ist. An einem distalen Ende 34 steht ein Anschluss 36 vor, der so ausgebildet ist, dass in diesen der Zapfen 14 am proximalen Ende des Nahtaufsatzes 10 eingeschoben werden kann. Über die Überwurfmutter 16, die mit einem Innengewinde versehen ist, kann dann eine feste Schraubverbindung mit dem Außengewinde 37 des Anschlusses 36 geschaffen werden.

Diese Verbindung kann aber auch andersartig ausgebildet sein, bspw. als Bajonettverbindung, als Schnappverbindung oder auch als dauerhafte feste Verbindung, wenn das chirurgische Nahtinstrument 30 nur mit einem einzigen Nahtaufsatz mit einer ganz bestimmten ausgestalteten Hohlnadel verwendet werden soll. Üblicherweise wird allerdings das chirurgische Nahtinstrument 30 mit einem ganzen Satz an unterschiedlichen Nahtaufsätzen angeboten, wie das aus dem eingangs erwähnten Katalog der Anmelderin ersichtlich ist.

Durch den stabförmigen Körper 32 des chirurgischen Nahtinstrumentes 30 kann von proximal nach distal ein chirurgischer Faden 40 hindurchgefädelt werden, wobei ein Abschnitt des Fadens im Bereich einer Mulde 38 im stabförmigen Körper 32 freiliegend ist. Eine Handhabungsperson, die den stabförmigen Körper 32 in der Hand hält, kann den Faden 40 bspw. mit einem Daumen, der in die Mulde 38 eingreift, hin und her bewegen.

Der Faden 40 wird über den hohlen Zapfen 14 des Anschlusses 12 des Nahtaufsatzes 10 in das Innere des Körpers 18 eingeführt, durch diesen hindurchgeführt und tritt an der Spitze 24 der gekrümmten Hohlnadel 22 aus, wie das in Fig. 1 dargestellt ist.

Aus Fig. 1 ist auch zu erkennen, dass der Faden 40 ein distales versteiftes Ende 42 aufweist.

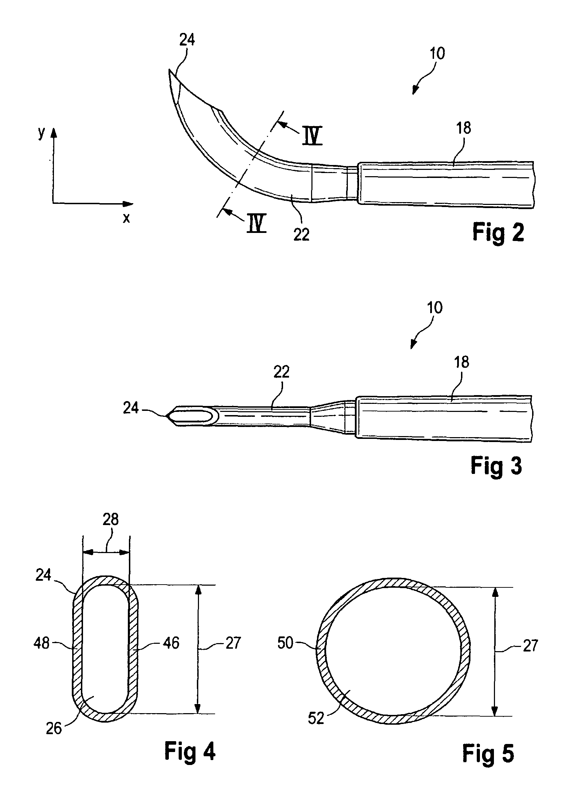

Um dieses versteifte Ende 42 um die Krümmung der gekrümmten Hohlnadel 22 führen zu können, ist, wie das insbesondere aus Fig. 4 ersichtlich ist, der Querschnitt 26 der Hohlnadel 22 ovalär. Das Oval ist als Flachoval ausgebildet, das heißt, die beiden gegenüberliegenden Seiten 46 und 48 sind flach bzw. verlaufen, wie ersichtlich, geradlinig und gehen jeweils über halbkreisförmige Krümmungen ineinander über.

Der ovale Querschnitt 26 weist eine längere Achse 27 und eine kürzere Achse 28 auf. Das hier dargestellte Längenverhältnis ist etwa 3:1.

Die Ausrichtung der längeren Achse 27 ist so, dass diese sich in der Ebene der Krümmung der Hohlnadel 22 erstreckt, wie das in Fig. 2 durch die Ebenenkoordinaten x und y dargestellt ist. Aus der Draufsicht von Fig. 3 ist zu erkennen, dass die gekrümmte Hohlnadel 22 hier schmaler ausgebildet ist als in der in Fig. 2 ersichtlichen Seitenansicht.

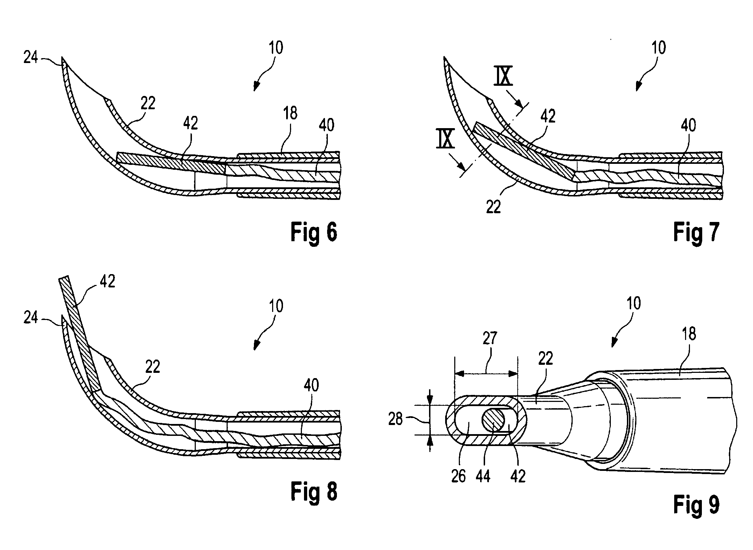

Der besondere Vorteil der Erfindung soll nunmehr anhand der Figurenfolge von Fig. 6 bis 8 beschrieben werden.

Wie bereits erwähnt, weist der Faden 40 einen versteiften Endabschnitt 42 auf. Dieses versteifte Ende ist problemlos durch den sich geradlinig erstreckenden, stabförmigen Körper 32 des Nahtinstrumentes 30 und durch den sich geradlinig erstreckenden, hohlschaftartigen Körper 18 des Nahtaufsatzes 10 hindurchzuführen. Durch die Ausgestaltung des ovalären Querschnitts 26 (siehe Fig. 4) steht nun dem versteiften Abschnitt 42 im Bereich der gekrümmten Hohlnadel 22 ausreichend Platz zur Verfügung, um den gekrümmten Bereich zu durchlaufen, wie das aus der Figurenfolgen von Fig., 6 zu Fig. 7 zu Fig. 8 ersichtlich ist. Es ist zu erkennen, dass der versteifte Abschnitt 42 bieg- und verkantfrei durch die Krümmung der Hohlnadel 22 geschoben werden kann.

Aus der Schnittdarstellung von Fig. 9 ist ersichtlich, dass das Maß der kürzeren Achse 28 des ovalären Querschnitts 26 in etwa dem Außendurchmesser 44 des versteiften Endes 42 entspricht.

Das Längenmaß der längeren Achse 27 und der Krümmungsradius der Krümmung bestimmen das Längenmaß des versteiften Endes 42, das hindurchgeschoben werden kann oder entsprechend umgekehrt, je nach dem, auf welche Bedingung Rücksicht genommen werden muss. Aus der Schnittdarstellung von Fig. 9 ist auch zu erkennen, dass in seitlicher Richtung, also in Erstreckung der kürzeren Achse 28 das versteifte Fadenende 42 seitlich durch die beiden flachen Seiten 46 und 48 des Flachovales geführt ist. Dadurch ist ein Ausweichen oder ein Verkanten in dieser Richtung ausgeschlossen.

Aus dem Vergleich von Fig. 4 und Fig. 5 ist ein weiterer Vorteil der Erfindung zu erkennen. Wie zuvor beschrieben, eröffnet das Maß der längeren Achse 27 des ovalären Querschnitts 26 in Zusammenhang mit den flachen Seiten 46, 48 die einwandfrei geführte, verkantfreie Bewegung des versteiften Endes 42 durch die gekrümmte Hohlnadel hindurch. In Fig. 5 ist eine Hohlnadel mit kreisförmigem Querschnitt dargestellt, dessen Innendurchmesser dem Maß der längeren Achse 27 entspricht.

Durch eine solche gekrümmte Hohlnadel könnte das versteifte Ende 42 ebenfalls hindurchgeschoben werden, nur wäre der Hohlnadelkörper bzw. das Volumen des Hohlnadelkörpers wesentlich größer, so dass eine wesentlich größere Einstichstelle in das Gewebe resultieren würde.

Außerdem wäre nicht ausgeschlossen, dass sich der versteifte Endabschnitt 42 seitlich verkantet und ggf. verklemmt, denn er könnte ja auch seitlich ausweichen, was durch die Ausgestaltung, wie das insbesondere in Fig. 9 ersichtlich ist, verhindert wird.

Hier wird also nicht nur weniger Raum benötigt, so dass der Einstich in das Gewebe mit der Hohlnadel 22 weniger traumatisch durchzuführen ist, auch das Durchführen des versteiften Fadenendes 42 erfolgt wesentlich sicherer.

Aus dem Vergleich von Fig. 4 und Fig. 5 ist auch ein weiterer Vorteil zu entnehmen, nämlich, dass durch die Ausbildung als Flachoval die flachen Seiten 46 und 48 wesentlich höhere Verformungskräfte aufnehmen können als der in Fig. 5 gezeigte Körper. Diese Kräfte werden als Widerstandskräfte beim Einstechen dahingehend wirken, dass sich die Krümmung etwas aufweitet, das heißt, diese Kräfte wirken im Wesentlichen in Richtung der längeren Achse 27.

Da diese Kräfte aufgrund der Geometrie wesentlich besser aufgenommen werden können, ergibt sich die Möglichkeit, die Wandstärke des Flachovals zu reduzieren.

In dem vorangegangenen Ausführungsbeispiel wurde ein Faden in Form eines Monofilamentes dargestellt, der ein versteiftes Ende 42 aufweist.

Die Erfindung wirkt sich besonders günstig auch bei Multifilamentfäden auf, deren distale Enden über eine Hülse oder einem entsprechenden verklebten Ende zusammengebündelt sind. Ein solcher Zusammenbau kann ebenfalls einfach durch die gekrümmte Hohlnadel 22 hindurchbewegt werden.

## Patentansprüche

1. Nahtaufsatz für ein chirurgisches Instrument (30), mit einem proximalseitigen Anschluss (12) zum Verbinden mit dem chirurgischen Nahtinstrument (30), mit einem hohlschaftartigen Körper (18), der distalseitig (20) eine gekrümmte Hohlnadel (22) aufweist, wobei ein Faden (40) oder ein Fadenbündel durch den Nahtaufsatz (10) führbar ist und ein Querschnitt (26) der gekrümmten Hohlnadel (22) ovalär ist, **dadurch gekennzeichnet, dass** der Querschnitt (26) als Flachoval ausgebildet ist, dessen beide gegenüberliegende Seiten (46 und 48) flach verlaufen, und dass sich eine längere Achse (27) des Flachovales in einer Krümmungsebene (x, y) der gekrümmten Hohlnadel (22) erstreckt.

2. Nahtaufsatz nach Anspruch 1, **dadurch gekennzeichnet, dass** die kürzere Achse (28) des Flachovales in etwa dem Durchmesser (44) des versteiften distalen Endes des Fadens (40) oder des Fadenbündels entspricht.

3. Chirurgisches Nahtinstrument mit einem etwa stabförmigen Körper (32), durch den ein chirurgischer Faden (40) oder ein Fadenbündel von proximal nach distal führbar ist, **dadurch gekennzeichnet, dass** der Körper (32) mit einem Nahtaufsatz (10) nach einem der Ansprüche 1 oder 2 verbunden ist.

## Claims

1. Needle attachment for a surgical instrument (30), having a proximal-side connector (12) for connecting to the surgical sewing instrument (30), with a hollow shaft-like body (18) which has a curved hollow needle (22) on the distal side (20), whereby a thread (40) or a thread bundle can be guided through the needle attachment (10), and wherein a cross section (26) of the curved hollow needle (22) is oval, **characterized in that** the cross section (26) is configured as a flat oval the opposite sides (46 and 48) of which are flat, and wherein a longer axis (27) of the flat oval extends in a bending plane (x, y) of the curved hollow needle (22).

2. Needle attachment of claim 1, **characterized in that** the shorter axis (28) of the flat oval corresponds approximately to the diameter (44) of the stiffened distal end of the thread (40) or of the thread bundle.

3. Surgical sewing instrument with an approximately rod-like body (32), through which a surgical thread (40) or a thread bundle can be guided from proximal to distal, **characterized in that** the body (32) is connected to a needle attachment of claims 1 or 2.

## Revendications

1. Accessoire de suture pour un instrument chirurgical (30), avec un raccord (12) du côté proximal pour la liaison avec l'instrument chirurgical de suture (30), avec un corps (18) du genre tige creuse qui présente une aiguille creuse courbe (22) du côté distal (20), un fil (40) ou un faisceau de fils pouvant être guidé à travers l'accessoire de suture (10) et une section transversale (26) de l'aiguille creuse courbe (22) étant ovale, **caractérisé en ce que** la section transversale (26) est réalisée sous la forme d'un ovale plat dont les deux côtés opposés (46 et 48) sont plats, et qu'un axe long (27) de l'ovale plat s'étend dans un plan de courbure (x, y) de l'aiguille creuse courbe (22).

2. Accessoire de suture selon la revendication 1, **caractérisé en ce que** l'axe court (28) de l'ovale plat correspond approximativement au diamètre (44) de l'extrémité distale rigidifiée du fil (40) ou du faisceau de fils.

3. Instrument chirurgical de suture avec un corps (32) approximativement en forme de tige, à travers lequel un fil chirurgical (40) ou un faisceau de fils peut être guidé de proximal vers distal, **caractérisé en ce que** le corps (32) est relié à un accessoire de suture (10) selon une des revendications 1 ou 2.
